(19) Europäisches Patentamt
European Patent Office
Office européen des brevets

(11) Numéro de publication: **0 037 781**
**B1**

# (12) FASCICULE DE BREVET EUROPEEN

(45) Date de publication du fascicule du brevet: **11.01.84**

(51) Int. Cl.³: **C 07 C 93/02**, A 61 K 31/13

(21) Numéro de dépôt: **81400533.6**

(22) Date de dépôt: **02.04.81**

(54) **Phényl alcoxy propanolamines, leur préparation et leur application en tant que médicaments.**

(30) Priorité: **04.04.80 FR 8007717**

(43) Date de publication de la demande: **14.10.81 Bulletin 81/41**

(45) Mention de la délivrance du brevet: **11.01.84 Bulletin 84/2**

(84) Etats contractants désignés: **AT BE CH DE FR GB IT LI LU NL SE**

(56) Documents cités:
**BE - A - 699 789**
**DD - A - 56 533**

**DIE PHARMAZIE, vol. 26, no. 8, août 1971 H. SCHULZ et al.: "Darstellung von herz- und kreislaufwirksamen Verbindungen", pages 477-481**
**CHEMICAL ABSTRACTS, vol. 83, no. 7, 18 août 1975, page 464, abrégé 58414t Columbus, Ohio, US**

(73) Titulaire: **PIERRE FABRE S.A., 125, rue de la Faisanderie, F-75116 Paris (FR)**

(72) Inventeur: **Mouzin, Gilbert, Dr. Chim., 21, rue Sainte-Foy, F-81100 Castres (FR)**
Inventeur: **Cousse, Henri, Dr. Chim., La Foun de los Nobios Chemin de Lastinos, F-81100 Castres (FR)**
Inventeur: **Vilain, Pol, Les Gaux, F-81290 Labrugiere (FR)**

(74) Mandataire: **Corre, Jacques Denis Paul et al, Cabinet Regimbeau 26, Avenue Kléber, F-75116 Paris (FR)**

**EP 0 037 781 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Phenyl alcoxy propanolamines, leur préparation et leur application en tant que médicaments

La présente invention, réalisée au Centre de Recherches Pierre FABRE, a pour objet de nouveaux dérivés de phényl alcoxy propanolamines, leur procédé de fabrication et leur application en thérapeutique, notamment dans le traitement de l'hypertension et de l'angor.

L'invention vise également les compostitions pharmaceutiques contenant ces dérivés utilisés comme principes actifs.

Les principes actifs actuellement commercialisés, possédant une activité béta-bloquante, présentent la structure générale:

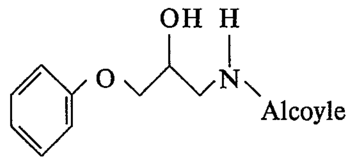

Ce sont des éthers de phénols, il était donc imprévisible que les dérivés éthers d'alcools présentent le même type d'activité béta-bloquante.

Pour illustrer l'arrière plan technologique de l'objet de la présente invention, on mentionnera par exemple les documents de technique antérieure suivants: Die Pharmazie, vol. 26, n° 8, août 1971, H. Schulz et al.: Darstellung von herz- und kreislaufwirksamen Verbindungen, page 477—481, page 477 et tables 4—5; DD-A-56 533 (Schulz, Jassmann et Forster); BE-A-699 789 (VEB Fahlberg-List Magdeburg, chemische und pharmazeutische Fabriken); Chemicals Asstracts, vol. 83, n° 7, 18 août 1975, page 464, abrégé 58 414 t Colombus Ohio US et JP-A-7 512 039 (Tanable Seiyaku Co LTD) 07-02-1975, abrégé.

La présente invention a pour objet de nouveaux dérivés de phényl alcoxy propanolamines de formule générale I:

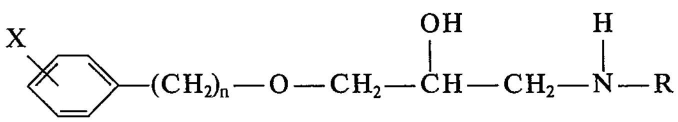

(I)

dans laquelle:

X représente un hydrogène, un radical alcoyle inférieur et plus particulièrement le radical méthyle, un radical alcoxy inférieur et plus particulièrement le radical méthoxy,

n représente les valeurs 1 à 3, et

R représente un radical isopropyle ou terbutyle,

ainsi que leurs sels avec les acides minéraux ou organiques thérapeutiquement acceptables.

Les acides minéraux ou organiques susceptibles de donner des sels thérapeutiquement acceptables avec les composés de formule générale I, sont par exemple l'acide chlorhydrique, l'acide sulfurique, l'acide phosphorique, l'acide succinique, l'acide oxalique, l'acide tartrique, l'acide maléique ou l'acide fumarique.

Dans le cadre de la présente invention on entendra par alcoyle inférieur un radical alcoyle contenant de 1 à 8 et de préférence de 1 à 4 atomes de carbone.

Ces dérivés sont doués d'activités béta-bloquantes, anti-arythmique, anti-calcium qui sont utiles dans le traitement de diverses maladies d'origine cardiaque.

L'invention concerne également un procédé de préparation des composés de formule générale I par réaction d'un glycidyl éther de formule II

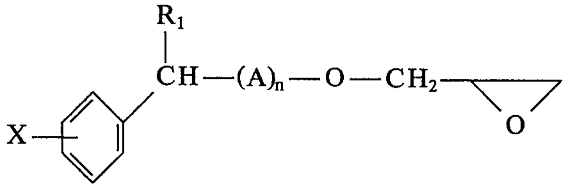

(II)

sur une amine primaire de formule III

$$R-NH_2 \qquad (III)$$

dans un solvant, en particulier un alcool tel que le méthanol.

Conformément à la présente invention, le glycidyl éther de formule II peut être préparé par de l'épichlorhydrine sur un alcool de formule IV

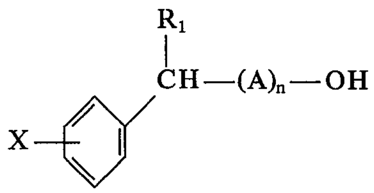

(IV)

en présence d'une base, telle que la soude, et d'un catalyseur du type ammonium quaternaire, tel qu'un composé de formule

$^{\oplus}N(R_0)_4 \quad X^{\ominus}$

dans laquelle $R_0$ est un radical alcoyle inférieur et $X^{\ominus}$ l'anion d'un acide, par exemple $HSO_3^{\ominus}$ ou $Hal^{\ominus}$, tel qu'en particulier l'hydrogénosulfate de tétrabutylammonium.

Dans les formules II, III et IV les différents radicaux ont la même signification que les radicaux correspondants de la formule générale I.

On notera que les différents composés de formules III et IV sont connus dans la technique antérieure et/ou peuvent être facilement obtenus par des procédés en soi connus.

Les composés chimiques suivants et leur mode de préparation sont cités à titre d'exemples non limitatifs.

## Exemple 1

### Préparation de l'isopropylamino-3, m-tolyl méthoxy-1 propanol-2 fumarate

#### a) Préparation du méta méthyl benzyloxy-1, époxy-2-3 propane

A un milieu hétérogène de 50 ml d'épichlorhydrine, 50 ml de soude à 50% et 1,36 g d'hydrogénosulfate de tétrabutylammonium, on introduit en 20 minutes 12,2 g (0,1 mole) de métaméthylbenzylalcool, en refroidissant sur bain de glace.

Après deux heures sous forte agitation, on ajoute 100 ml d'eau et on extrait deux fois à l'éther. La phase organique est lavée avec une solution bicarbonatée, puis à l'eau et on sèche sur sulfate de sodium.

Après filtration et évaporation du solvant, on obtient avec un rendement quantitatif l'époxyde intermédiaire.

#### b) préparation de l'isopropyl-3 métatolylméthoxy-1 propanol-2 fumarate

A une solution de 10 g de métaméthylbenzyloxy-1 époxy-2-3 propane (0,0056 mole) dans 100 ml de méthanol absolu, refroidie par un bain de glace, on ajoute sous agitation 17 ml (11,82 g ou 0,2 mole) d'isopropylamine. On laisse une nuit à température ambiante sous forte agitation, et on évapore jusqu'à siccité.

L'huile résiduelle est reprise par l'éther, on lave à l'eau bicarbonatée puis à l'eau saturée de chlorure de sodium.

La solution éthérée est séchée sur sulfate de sodium, après filtration on traite par 3,65 g (0,03 mole) d'acide fumarique, on récupère après filtration et séchage 83% de produit de formule:

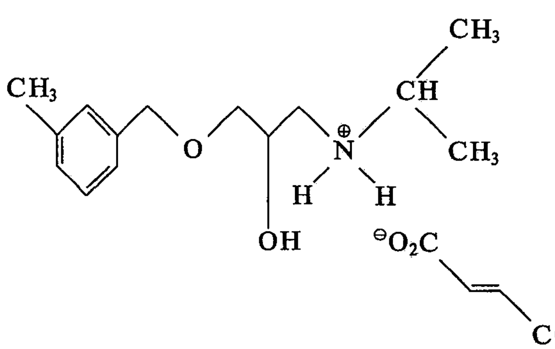

Formule brute: $C_{18}H_{27}NO_6$
Masse moléculaire: 353,4
Cristaux: blancs
Point de fusion: 104° C
Spectre de RMN (DMSO-$d_6$) $\delta$ ppm: 1,25 (d, 6H); 2,3 (s, 3H); 2,7 à 3,7 (m, 5H); 4 (m, 1H); 4,45 (s, 2H); 6,55 (s, 2H); 7,1 (s, 4H); 9,2 (s, 2H) échangeables.
Chromatographie sur plaque:
— support: gel de silice 60 F 254 Merck
— solvant: chloroforme — méthanol — ammoniaque 80/18/2
— révélation: 0,5
— Rf: 0,5
Solubilités: Soluble dans l'eau à 10% et à 15% dans le diméthylsulfoxyde.

Exemple 2

Préparation du tertiobutylamino-3 métatolylméthoxy-1 propanol-2 maléate

D'une façon similaire à celle décrite dans l'exemple 1, mais en utilisant la tertiobutylamine et l'acide maléique comme agent salifiant, on obtient le produit de formule:

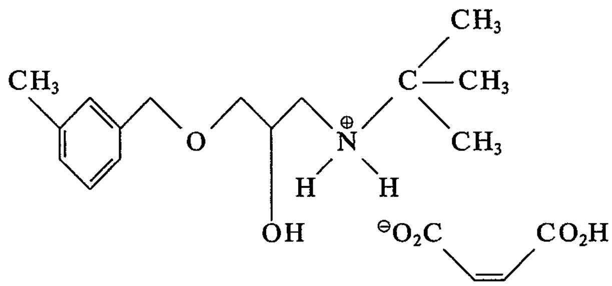

Formule brute: $C_{19}H_{29}NO_6$
Masse moléculaire: 367,4
Cristaux: blancs
Point de fusion: 108° C
Spectre RMN (DMSO-$d_6$): $\delta$ ppm: 1,3 (s, 9H); 2,3 (s, 3H); 2,4 à 3,3 (m, 2H); 3,5 (d, 2H); 4 (m, 1H); 4,5 (s, 2H); 6,15 (s, 2H); 7,15 (s, 4H).
Chromatographie sur plaque:
— support: gel de silice 60 F 254 Merck
— solvant: chloroforme — méthanol — ammoniaque 80/18/2
— révélation: UV et iode
— Rf: 0,5
Solubilités: Soluble dans l'eau à 2,5% et à 10% dans le diméthylsulfoxyde.

Exemple 3

Préparation de l'isopropylamino-3 (orthométhoxy benzyloxy)-1 propanol-2 fumarate

D'une manière similaire à celle décrite dans l'exemple 1, mais en utilisant l'orthométhoxy benzylalcool, on obtient le produit de formule:

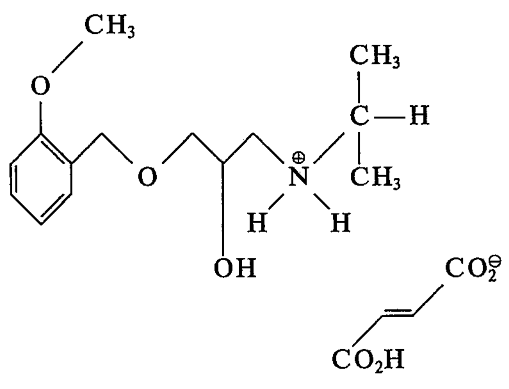

Formule brute: $C_{18}H_{27}N\ O_7$
Masse moléculaire: 369,4
Cristaux: blancs
Point de fusion: 110° C
Chromatographie sur plaque:
- support: gel de silice 60 F 254 Merck
- solvant: chloroforme — méthanol — ammoniaque 80/18/2
- révélation: UV et iode
- Rf: 0,50

Solubilités: Soluble dans l'eau à 10% et dans le diméthylsulfoxyde à 15%.

## Exemple 4

### Préparation du tertiobutylamino-3 (orthométhoxy benzyloxy)-1 propanol-2 maléate

D'une façon similaire à celle décrite dans l'exemple 3, mais en utilisant la tertiobutylamine et l'acide maléique comme agent salifiant, on obtient le produit de formule:

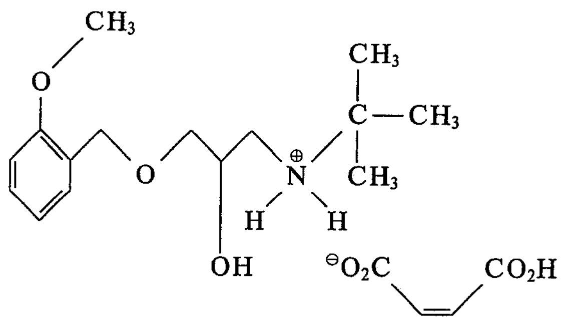

Formule brute: $C_{19}H_{29}N\ O_7$
Masse moléculaire: 383,4
Cristaux: blancs
Point de fusion: 104° C
Chromatographie sur plaque:
- support: gel de silice 60 F 254 Merck
- solvant: méthanol — chloroforme — ammoniaque 80/18/2
- révélation: UV et iode
- Rf: 0,54

Solubilités: Soluble dans l'eau à 10%, soluble dans le diméthylsulfoxyde à 15%.

## Exemple 5

### Préparation du tertiobutylamino-3 (métaméthoxybenzyloxy)-1 propanol-2 maléate

D'une façon similaire à celle décrite dans l'exemple 1, mais en utilisant le métaméthoxy benzyl alcool, la tertiobutylamine et l'acide maléique comme agent salifiant, on obtient le produit de formule:

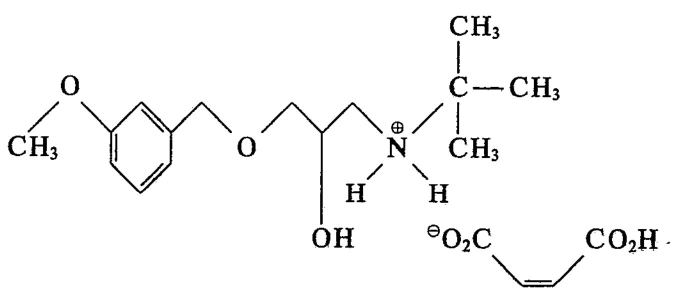

Formule brute: $C_{19}H_{29}N\ O_7$
Masse moléculaire: 383,4
Cristaux: blancs
Point de fusion: 89° C

Chromatographie sur plaque:
- support: gel de silice 60 F 254 Merck
- solvant: chloroforme — méthanol — ammoniaque 80/18/2
- révélation: UV et iode
- Rf: 0,65

Solubilités: Soluble dans l'eau à 10%, soluble dans le diméthylsulfoxyde à 12%.

Exemple 6

Préparation du tertiobutylamino-3 (paraméthoxy benzyloxy)-1 propanol-2 maléate

D'une façon similaire à celle décrite dans l'exemple 1, mais en utilisant le paraméthoxy benzylalcool, la tertiobutylamine et l'acide maléique comme agent salifiant, on obtient le produit de formule:

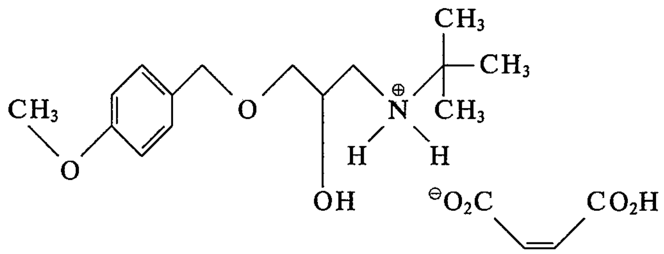

Formule brute: $C_{19}H_{29}N\ O_7$
Masse moléculaire: 383,4
Cristaux: blancs
Point de fusion: 102° C
Chromatographie sur plaque:
- support: gel de silice 60 F 254 Merck
- solvant: chloroforme — méthanol — ammoniaque 80/18/2
- révélation: UV et iode
- Rf: 0,63

Solubilités: Soluble dans l'eau à 10%, soluble dans le diméthylsulfoxyde à 20%.

Exemple 7

Préparation de l'isopropylamino-3 (phényl-3 propoxy)-1 propanol-2 fumarate

D'une façon similaire à celle décrite dans l'exemple 1, mais en utilisant le phényl-3 propanol-1, on obtient le produit de formule:

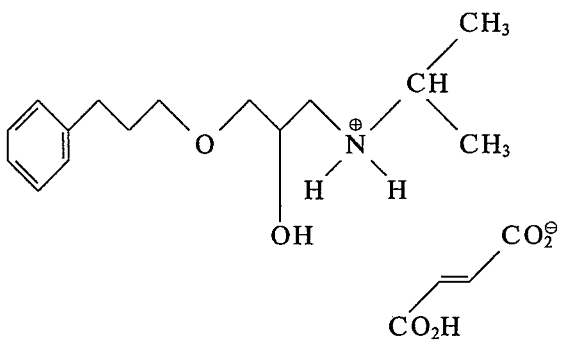

Formule brute: $C_{19}H_{29}N\ O_6$
Masse moléculaire: 367,4
Cristaux: blancs
Point de fusion: 86,5° C
Chromatographie sur plaque:
- support: gel de silice 60 F 254 Merck
- solvant: chloroforme — méthanol — ammoniaque 80/18/2
- révélation: UV et iode
- Rf: 0,50

Solubilités: Soluble dans l'eau à 10%, et dans le diméthylsulfoxyde à 12%.

Exemple 8

Préparation du tertiobutylamino-3 (phényl-3 propoxy)-1 propanol-2 fumarate

D'une façon similaire à celle décrite dans l'exemple 1, mais en utilisant le phényl-3 propanol-1 et la tertiobutylamine, on obtient le produit de formule:

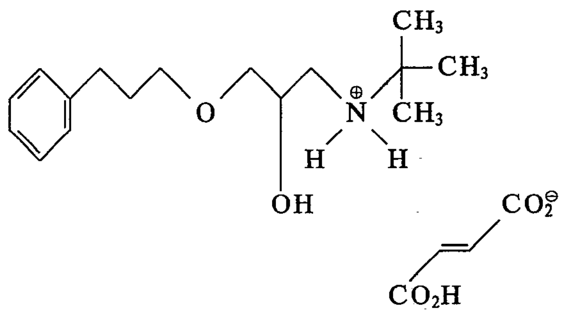

Formule brute: $C_{20}H_{31}NO_6$
Masse moléculaire: 381,4
Cristaux: blancs
Point de fusion: 90° C
Chromatcgraphie sur plaque:
— support: gel de silice 60 F 254 Merck
— solvant: chloroforme — méthanol — ammoniaque 80/18/2
— révélation: UV et iode
— Rf: 0,50
Solubilités: Soluble à 10% dans l'eau, à 15% dans le diméthylsulfoxyde.

Les propriétés béta-bloquantes des composés de formule générale I ont pu être mises en évidence à l'aide de l'expérimentation suivante.

Experimentation

A) Toxicologie

Les composés chimiques précédemment décrits ont été soumis à des contrôles de toxicité. L'étude de la toxicité a été effectuée chez la souris conventionnelle pesant de 20 à 22 grammes. Les substances ont été administrées par voie intraveineuse et orale.

Les $DL_{50}$ sont calculées selon la méthode de KÄRBER G. — Arch. Exptl. Pathol. Pharmacol., 1931, 162, 480.

Par voie intraveineuse les $DL_{50}$ sont comprises entre 25 et 100 mg/kg. Par voie orale les $DL_{50}$ sont comprises entre 600 et 1200 mg/kg.

B) Etude pharmacologique

Les expérimentations pharmacologiques auxquelles ont été soumis les composés chimiques objet de l'invention ont permis de mettre en évidence d'intéressantes propriétés béta-bloquantes.

Méthode

Tachycardie isoprénalique chez le chien (DUNLOP O. et SHANK P. — Brit. J. Pharmacol., 1968, 32, 201 à 218).

Les produits les plus intéressants réduisent significativement la tachycardie isoprénalique à partir de la dose de 1 mg/kg par voie intraveineuse.

C) Applications thérapeutiques

Compte tenu de leur propriété béta-bloquante et de leur faible toxicité, ces composés peuvent être utilisés en thérapeutiques et plus particulièrement dans le traitement de l'hypertension.

Ces composés peuvent être utilisés sous la forme de compositions pharmaceutiques dans lesquelles on mélange le principe actif avec des diluants ou véhicules non toxiques

pharmaceutiquement acceptables. Ces compositions peuvent être administrées par voie orale, parentérale, intraveineuse, ou rectale.

Les compositions peuvent être par exemple sous la forme de comprimés, de capsules, de suppositoires, de solutions aqueuses ou huileuses, de suspensions aqueuses ou huileuses, d'émulsions, de poudres dispersables ou de solutions ou suspensions aqueuses ou huileuses injectables.

Les compositions pharmaceutiques selon l'invention peuvent contenir, outre les composés de formule générale I, d'autres principes actifs venant compléter ou renforcer leurs aktions thérapeutiques.

**Revendications pour les Etats contractants: BE, CH, DE, FR, GB, IT, LI, LU, NL, SE**

1. Nouveaux dérivés de phényl alcoxy propanolamines répondant à la formule générale I:

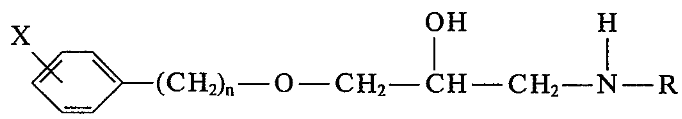

(I)

dans laquelle:

X représente un hydrogène, un radical alcoyle inférieur et plus particulièrement le radical méthyle, un radical alcoxy inférieur et plus particulièrement le radical méthoxy,
n représente les valeurs 1 à 3, et
R représente un radical isopropyle ou terbutyle,

ainsi que leurs sels avec les acides minéraux ou organiques thérapeutiquement acceptables.

2. L'isopropylamino-3 méta tolyl méthoxy-1 propanol-2 fumarate de formule I selon la revendication 1.

3. Le tertiobutylamino-3 métatolyl méthoxy-1 propanol-2 maléate de formule I selon la revendication 1.

4. L'isopropylamino-3 (ortho méthoxy benzyloxy)-1 propanol-2 fumarate de formule I selon la revendication 1.

5. Le tertiobutylamino-3 (ortho méthoxy benzyloxy)-1 propanol-2 maléate de formule I selon la revendication 1.

6. Le tertiobutylamino-3 (métaméthoxy benzyloxy)-1 propanol-2 maléate de formule I selon la revendication 1.

7. Le tertiobutylamino-3 (p-méthoxy benzyloxy)-1 propanol-2 maléate de formule I selon la revendication 1.

8. L'isopropylamino-3 (phényl-3 propoxy)-1 propanol-2 fumarate de formule I selon la revendication 1.

9. Le tertiobutylamino-3 (phényl-3 propoxy)-1 propanol-2 fumarate de formule I selon la revendication 1.

10. Procédé de préparation des composés de formule générale I, selon l'une des revendications 1 à 9, caractérisé en ce que l'on fait réagir un glycidyl éther de formule II:

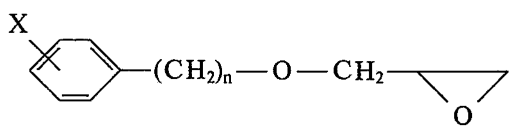

(II)

dans laquelle X et n ont les significations données à la revendication 1,

dans un milieu solvant, en particulier un alcool tel que le méthanol, sur une amine primaire de formule III:

$$R-NH_2 \qquad (III)$$

dans laquelle R a la signification donnée à la revendication 1.

11. Procédé selon la revendication 10, caractérisé en ce que le glycidyl éther de formule II est préparé par réaction, en présence d'une base et d'un catalyseur, de l'épichlorhydrine sur un alcool de formule IV:

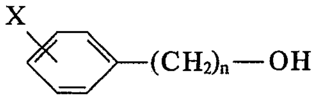

(IV)

dans laquelle X et n ont les significations données à la revendication 1.

12. Procédé selon la revendication 11, caractérisé en ce que le catalyseur est un composé d'ammonium quaternaire de formule générale:

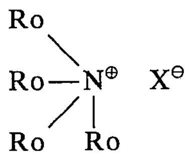

dans laquelle:

Ro représente un alcoyle, et
X représente $HSO_3^-$ ou $Hal^-$.

13. Procédé selon la revendication 12, caractérisé en ce que le composé d'ammonium quaternaire est l'hydrogénosulfate de tétrabutylammonium.

14. Procédé selon la revendication 11, caractérisé en ce que la base utilisée est une base minérale et plus particulièrement la soude.

15. A titre de médicaments nouveaux les composés selon l'une des revendications 1 à 9.

16. Les compositions pharmaceutiques caractérisées en ce qu'elles contiennent comme principe actif, au moins un composé selon l'une des revendications 1 à 9.

**Revendications pour l'Etat contractant: AT**

1. Procédé de préparation des composés de formule générale I:

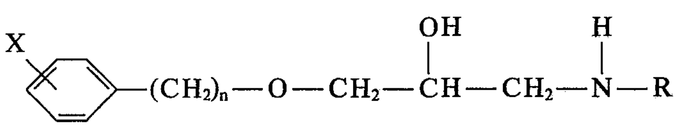

(I)

dans laquelle:

X représente un hydrogène, un radical alcoyle inférieur et plus particulièrement le radical méthyle, un radical alcoxy inférieur et plus particulièrement le radical méthoxy,
n représente les valeurs 1 à 3, et
R représente un radical isopropyle ou terbutyle,

ainsi que leurs sels avec les acides minéraux ou organiques thérapeutiquement acceptables,

caractérisé en ce que l'on fait réagir un glycidyl éther de formule II:

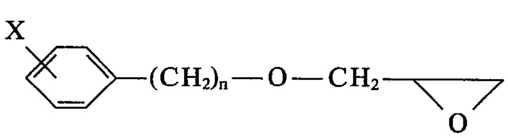

(II)

dans laquelle X et n ont les significations données ci-dessus, dans un milieu solvant, en particulier un alcool tel que le méthanol, sur une amine primaire de formule III:

$R-NH_2$ (III)

dans laquelle R a la signification donnée précédemment.

2. Procédé selon la revendication 1, caractérisé en ce que le glycidyl éther de formule II est préparé par réaction, en présence d'une base et d'un catalyseur, de l'épichlorhydrine sur un alcool de formule IV:

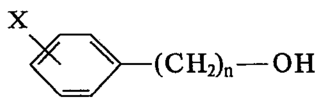

(IV)

dans laquelle X et n ont les significations données à la revendication 1.

3. Procédé selon la revendication 2, caractérisé en ce que le catalyseur est un composé d'ammonium quaternaire de formule générale:

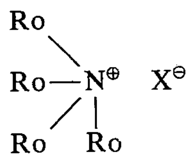

dans laquelle:

Ro représente un alcoyle, et
X représente $HSO_3^-$ ou $Hal^-$.

4. Procédé selon la revendication 3, caractérisé en ce que le composé d'ammonium quaternaire est l'hydrogénosulfate de tétrabutylammonium.

5. Procédé selon la revendication 2, caractérisé en ce que la base utilisée est une base minérale et plus particulièrement la soude.

6. Procédé selon l'une des revendications 1 à 5, caractérisé en ce que l'on prépare l'isopropylamino-3 méta tolyl méthoxy-1 propanol-2 fumarate.

7. Procédé selon l'une des revendications 1 à 5, caractérisé en ce que l'on prépare le tertiobutylamino-3 métatolyl méthoxy-1 propanol-2 maléate.

8. Procédé selon l'une des revendications 1 à 5, caractérisé en ce que l'on prépare l'isopropylamino-3 (ortho méthoxy benzyloxy)-1 propanol-2 fumarate.

9. Procédé selon l'une des revendications 1 à 5, caractérisé en ce que l'on prépare le tertiobutylamino-3 (ortho méthoxy benzyloxy)-1 propanol-2 maléate.

10. Procédé selon l'une des revendications 1 à 5, caractérisé en ce que l'on prépare le tertiobutylamino-3 (métaméthoxy benzyloxy)-1 propanol-2 maléate.

11. Procédé selon l'une des revendications 1 à 5, caractérisé en ce que l'on prépare le tertiobutylamino-3 (p-méthoxy benzyloxy)-1 propanol-2 maléate.

12. Procédé selon l'une des revendications 1 à 5, caractérisé en ce que l'on prépare l'isopropylamino-3 (phényl-3 propoxy)-1 propanol-2 fumarate.

13. Procédé selon l'une des revendications 1 à 5, caractérisé en ce que l'on prépare le tertiobutylamino-3 (phényl-3 propoxy)-1 propanol-2 fumarate.

**Patentansprüche für die Vertragsstaaten: BE, CH, DE, FR, GB, IT, LI, LU, NL, SE**

1. Neue Phenyl-alkoxy-propanolaminderivate entsprechend der allgemeinen Formel I:

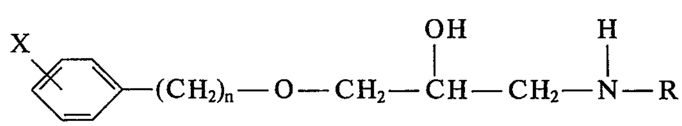

(I)

in welcher

X Wasserstoff, ein niedriges Alkoylradikal und insbesondere ein Methylradikal, ein niedriges Alkoxyradikal und insbesondere ein Methoxyradikal,
n die Werte 1 bis 3, und
R ein Isopropyl- oder Terbutylradikal darstellt,

sowie deren Salze mit therapeutisch annehmbaren Mineralsäuren oder organischen Säuren.

2. Isopropylamino-3 m-tolyl methoxy-1 propanol-2 fumarat der Formel I nach Anspruch 1.

3. Tert-Butylamino-3 m-tolyl methoxy-1 propanol-2 maleat der Formel I nach Anspruch 1.

4. Isopropylamino-3 (o-methoxy-benzyloxy)-1 propanol-2 fumarat der Formel I nach Anspruch 1.

5. Tert-Butylamino-3 (o-methoxy-benzyloxy)-1 propanol-2 maleat der Formel I nach Anspruch 1.

6. Tert-Butylamino-3 (m-methoxy-benzyloxy)-1 propanol-2 maleat der Formel I nach Anspruch 1.

7. Tert-Butylamino-3 (p-methoxy-benzyloxy)-1 propanol-2 maleat der Formel I nach Anspruch 1.

8. Isopropylamino-3 (phenyl-3 propoxy)-1 propanol-2 fumarat der Formel I nach Anspruch 1.

9. Tert-Butylamino-3 (phenyl-3 propoxy)-1 propanol-2 fumarat der Formel I nach Anspruch 1.

10. Verfahren zur Herstellung der Verbindungen der allgemeinen Formel I nach einem der Ansprüche 1 bis 9, dadurch gekennzeichnet, daß ein Glycidyläther der Formel II

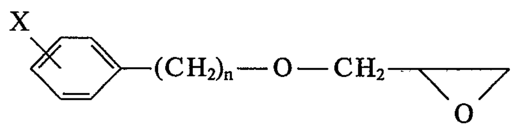

(II)

in welcher X und n die in Anspruch 1 angegebenen Bedeutungen haben,

in einem Lösungsmittel, insbesondere Alkohol, wie zum Beispiel Methanol, auf einem primären Amin der Formel III

$$R-NH_2 \qquad \text{(III)}$$

in welcher R die in Anspruch 1 angegebene Bedeutung hat, zur Reaktion gebracht wird.

11. Verfahren nach Anspruch 10, dadurch gekennzeichnet, daß der Glycidyläther der Formel II durch Reaktion, in Gegenwart einer Base und eines Katalysators, von Epichlorhydrin auf einem Alkohol der Formel IV

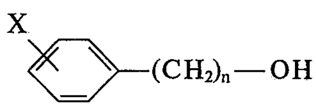

(IV)

in welcher X und n die in Anspruch 1 angegebenen Bedeutungen haben, hergestellt wird.

12. Verfahren nach Anspruch 11, dadurch gekennzeichnet, daß der Katalysator eine quaternäre Ammoniumverbindung der allgemeinen Formel

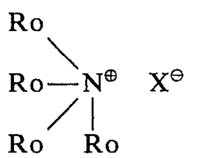

ist, in welcher

Ro ein Alkoyl und
X $HSO_3^-$ oder $Hal^-$

darstellt.

13. Verfahren nach Anspruch 12, dadurch gekennzeichnet, daß die quaternäre Ammoniumverbindung Tetrabutylammoniumhydrogensulfat ist.

14. Verfahren nach Anspruch 11, dadurch gekennzeichnet, daß die verwendete Base eine mineralische Base und insbesondere Soda ist.

15. Die Verbindungen nach einem der Ansprüche 1 bis 9 als neue Medikamente.

16. Die pharmazeutischen Zusammensetzungen, dadurch gekennzeichnet, daß sie als aktiven Hauptbestandteil zumindest eine Verbindung nach einem der Ansprüche 1 bis 9 enthalten.

**Patentansprüche für den Vertragsstaat: AT**

1. Verfahren zur Herstellung von Verbindungen der allgemeinen Formel I:

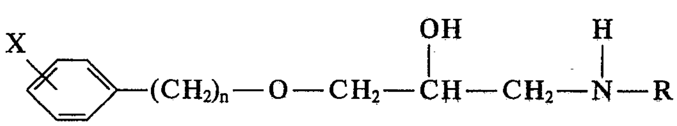

(I)

in welcher

X Wasserstoff, ein niedriges Alkoylradikal und insbesondere ein Methylradikal, ein niedriges Alkoxyradikal und insbesondere ein Methoxyradikal,
n die Werte 1 bis 3, und

R ein Isopropyl- oder Terbutylradikal darstellt,

sowie deren Salze mit therapeutisch annehmbaren Mineralsäuren oder organischen Säuren, dadurch gekennzeichnet, daß ein Glycidyläther der Formel II

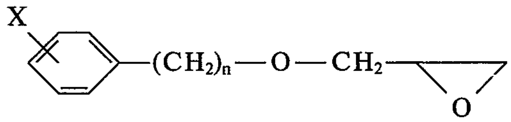

(II)

in welcher X und n die oben angegebenen Bedeutungen haben,

in einem Lösungsmittel, insbesondere Alkohol, wie zum Beispiel Methanol, auf einem primären Amin der Formel III

$$R-NH_2 \qquad (III)$$

in welcher R die vorher angegebene Bedeutung hat, zur Reaktion gebracht wird.

2. Verfahren nach Anspruch 1, dadurch gekennzeichnet, daß der Glycidyläther der Formel II durch Reaktion, in Gegenwart einer Base und eines Katalysators, von Epichlorhydrin auf einem Alkohol der Formel IV

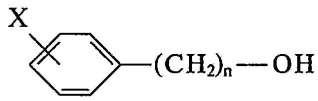

(IV)

in welcher X und n die in Anspruch 1 angegebenen Bedeutungen haben, hergestellt wird.

3. Verfahren nach Anspruch 2, dadurch gekennzeichnet, daß der Katalysator eine quaternäre Ammoniumverbindung der allgemeinen Formel

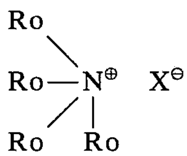

ist, in welcher

Ro ein Alkoyl und
X $HSO_3^-$ oder $Hal^-$

darstellt.

4. Verfahren nach Anspruch 3, dadurch gekennzeichnet, daß die quaternäre Ammoniumverbindung Tetrabutylammoniumhydrogensulfat ist.

5. Verfahren nach Anspruch 2, dadurch gekennzeichnet, daß die verwendete Base eine mineralische Base und insbesondere Soda ist.

6. Verfahren nach einem der Ansprüche 1 bis 5, dadurch gekennzeichnet, daß Isopropylamino-3 m-tolyl methoxy-1 propanol-2 fumarat hergestellt wird.

7. Verfahren nach einem der Ansprüche 1 bis 5, dadurch gekennzeichnet, daß tert-Butylamino-3 m-tolyl methoxy-1 propanol-2 maleat hergestellt wird.

8. Verfahren nach einem der Ansprüche 1 bis 5, dadurch gekennzeichnet, daß Isopropylamino-3 (o-methoxy benzyloxy)-1 propanol-2 fumarat hergestellt wird.

9. Verfahren nach einem der Ansprüche 1 bis 5, dadurch gekennzeichnet, daß tert-Butylamino-3 (o-methoxy benzyloxy)-1 propanol-2 maleat hergestellt wird.

10. Verfahren nach einem der Ansprüche 1 bis 5, dadurch gekennzeichnet, daß tert-Butylamino-3 (m-methoxy benzyloxy)-1 propanol-2 maleat hergestellt wird.

11. Verfahren nach einem der Ansprüche 1 bis 5, dadurch gekennzeichnet, daß tert-Butylamino-3 (p-methoxy benzyloxy)-1 propanol-2 maleat hergestellt wird.

12. Verfahren nach einem der Ansprüche 1 bis 5, dadurch gekennzeichnet, daß Isopropylamino-3 (phenyl-3 propoxy)-1 propanol-2 fumarat hergestellt wird.

13. Verfahren nach einem der Ansprüche 1 bis 5, dadurch gekennzeichnet, daß tert-Butylamino-3 (phenyl-3 propoxy)-1 propanol-2 fumarat hergestellt wird.

**Claims for the Contracting States: BE, CH, DE, FR, GB, IT, LI, LU, NL, SE**

1. Novel derivatives of phenyl alcoxy propanol amines having the general formula I:

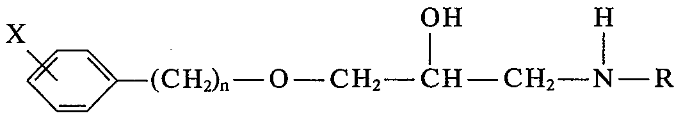

(I)

in which:

X represents a hydrogen atom, a lower alkyl radical and more particularly the methyl radical, a lower alcoxy radical and more particularly the methoxy radical,
n represents the values 1 – 3, and
R represents an isopropyl or terbutyl radical,

such that their salts with mineral or organic acids are therapeutically acceptable.

2. The 3-isopropylamino meta-tolyl 1-methoxy 2-propanol fumarate of the formula I according to claim 1.

3. The 3-tertiobutylamino meta-tolyl 1-methoxy 2-propanol maleate of the formula I according to claim I.

4. The 3-isopropylamino 1-(ortho-methoxy benzyloxy) 2-propanol fumarate of the formula I according to claim 1.

5. The 3-tertiobutylamino 1-(ortho-methoxy benzyloxy) 2-propanol maleate of the formula I according to claim 1.

6. The 3-tertiobutylamino 1-(meta-methoxy benzyloxy) 2-propanol maleate of the formula I according to claim 1.

7. The 3-tertiobutylamino 1-(para-methoxy benzyloxy) 2-propanol maleate of the formula I according to claim 1.

8. The 3-isopropylamino 1-(3-phenyl propoxy) 2-propanol fumarate of the formula I according to claim 1.

9. The 3-tertiobutylamino 1-(3-phenyl propoxy) 2-propanol fumarate of the formula I according to claim 1.

10. Process for preparing compounds of the general formula I, according to any of claims 1 to 9, characterised in that a glycidyl ether of the formula II:

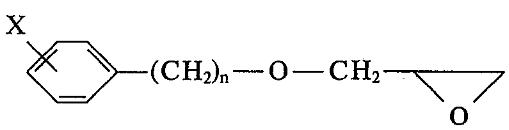

(II)

in which X and n have the significations given in claim 1, is reacted in a solvent medium, in particular in alcohol such as methanol, on the primary amine of the formula III:

$R-NH_2$ (III)

in which R has the signification given in claim 1.

11. Process according to claim 10, characterised in that the glycidyl ether of the formula II is prepared by reaction, in the presence of a base and a catalyst, of epichlorhydrin on an alcohol of the formula IV:

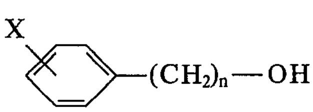

(IV)

in which X and n have the significations given in claim 1.

12. Process according to claim 11, characterised in that the catalyst is a quaternary ammonium compound of the general formula:

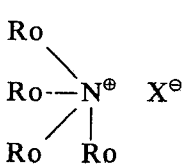

in which:

Ro represents an alkyl, and
X represents $HSO_3^-$ or $Hal^-$.

13. Prccess according to claim 12, characterised in that the quaternary ammonium compound is the hydrogen sulphate of tetrabutylammonium.

14. Process according to claim 11, characterised in that the base used is a mineral base and more particularly soda.

15. By way of new medicaments, the compounds according to any one of claims 1 to 9.

16. The pharmaceutical compositions characterised in that they contain as active constituent at least one compound according to any one of claims 1 to 9.

**Claims for the Contracting State: AT**

1. Process for preparing compounds of the general formula I:

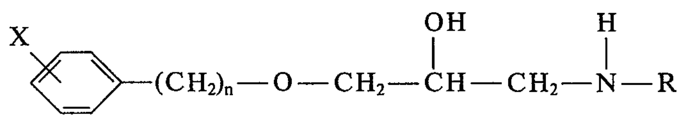

(I)

in which:

X represents a hydrogen atom, a lower alkyl radical and more particularly the methyl radical, a lower alcoxy radical and more particularly the methoxy radical,
n represents the values 1—3, and
R represents an isopropyl or terbutyl radical,
such that their salts with mineral or organic acids are therapeutically acceptable,

characterised in that a glycidyl ether of the formula II:

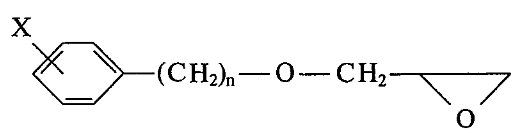

(II)

in which X and n have the significations given above, is reacted in a solvent medium, in particular an alcohol such as methanol, on a primary amine of the formula III:

$$R-NH_2 \qquad (III)$$

in which R has the signification given previously.

2. Process according to claim 1, characterised in that the glycidyl ether of the formula II is prepared by reaction, in the presence of a base and of a catalyst, of epichlorhydrin on an alcohol of the formula IV:

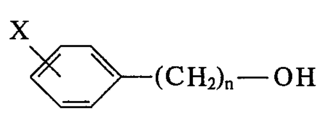

(IV)

in which X and n have the significations given in claim 1.

3. Process according to claim 2, characterised in that the catalyst is a quaternary ammonium compound of the general formula:

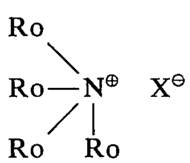

in which:

Ro represents an alkyl, and

X represents $HSO_3^-$ or $Hal^-$.

4. Process according to claim 3, characterised in that the quaternary ammonium compound is the hydrogen sulphate of tetrabutylammonium.
5. Process according to claim 2, characterised in that the base used is a mineral base and more particularly soda.
6. Process according to any one of claims 1 to 5, characterised in that the 3-isopropylamino meta-tolyl 1-methoxy 2-propanol fumarate of the formula I is prepared.
7. Process according to any one of claims 1 to 5, characterised in that the 3-tertiobutylamino meta-tolyl 1-methoxy 2-propanol maleate of the formula I is prepared.
8. Process according to any one of claims 1 to 5, characterised in that the 3-isopropylamino 1-(ortho-methoxy benzyloxy) 2-propanol fumarate of the formula I is prepared.
9. Process according to any of claims 1 to 5, characterised in that the 3-tertiobutylamino 1-(ortho-methoxy benzyloxy) 2-propanol maleate of the formula I is prepared.
10. Process according to any one of claims 1 to 5, characterised in that the 3-tertiobutylamino 1-(meta-methoxy benzyloxy) 2-propanol maleate of the formula I is prepared.
11. Process according to any one of claims 1 to 5, characterised in that the 3-tertiobutylamino 1-(para-methoxy benzyloxy) 2-propanol maleate of the formula I is prepared.
12. Process according to any one of claims 1 to 5, characterised in that the 3-isopropylamino 1-(3-phenyl propoxy) 2-propanol fumarate of the formula I is prepared.
13. Process according to any one of claims 1 to 5, characterised in that the 3-tertiobutylamino 1-(3-phenyl propoxy) 2-propanol fumarate of the formula I is prepared.